# EUROPEAN PATENT SPECIFICATION

(11) **EP 1 900 807 B1**
(45) Date of publication and mention of the grant of the patent: **26.02.2014**
(21) Application number: 07114680.7
(22) Date of filing: 21.08.2007
(51) Int. Cl.: C12N 1/02, C12M 1/26, B01L 3/00

(54) **Method of separating microorganism using nonplanar solid substrate and device for separating microorganism**
Verfahren zur Separierung von Mikroorganismen über nichtplanare Festsubstrate und Vorrichtung zur Separierung von Mikroorganismen
Procédé pour la séparation de micro-organismes utilisant un substrat solide non planaire et dispositif pour la séparation de micro-organismes

(30) Priority: 21.08.2006 KR 20060079053; 21.08.2006 KR 20060079056; 21.08.2006 KR 20060079055; 21.08.2006 KR 20060079054
(43) Date of publication of application: 19.03.2008
(73) Proprietor: Samsung Electronics Co., Ltd., Suwon-si, Gyeonggi-do, 443-742 (KR)
(72) Inventor: Hwang, Kyu-youn, Gyeonggi-do (KR); Jeong, Sung-young, Gyeonggi-do (KR); Kim, Joon-ho, Gyeonggi-do (KR); Lee, Hun-joo, Gyeonggi-do (KR); Han, Jung-im, Gyeonggi-do (KR)
(74) Representative: Grünecker, Kinkeldey, Stockmair & Schwanhäusser

(56) References cited:
- EP-A- 1 712 284
- EP-A- 1 726 641
- WO-A-98/51693
- WO-A-2005/093065
- US-A1- 2002 042 125
- ACARTURK T O ET AL: "Control of attachment, morphology, and proliferation of skeletal myoblasts on silanized glass." JOURNAL OF BIOMEDICAL MATERIALS RESEARCH 15 MAR 1999, vol. 44, no. 4, 15 March 1999 (1999-03-15), pages 355-370, XP002468347 ISSN: 0021-9304
- SPARGO ET AL: "Spatially controlled adhesion, spreading, and differentiation of endothelial cells on self-assembled molecular monolayers" PROCEEDINGS OF THE NATIONAL ACADEMY OF SCIENCES OF USA, NATIONAL ACADEMY OF SCIENCE, WASHINGTON, DC, US, vol. 91, November 1994 (1994-11), pages 11070-11074, XP002103614 ISSN: 0027-8424
- LIU QI-YING ET AL: "Synaptic connectivity in hippocampal neuronal networks cultured on micropatterned surfaces" DEVELOPMENTAL BRAIN RESEARCH, vol. 120, no. 2, 14 April 2000 (2000-04-14), pages 223-231, XP002468348 ISSN: 0165-3806
- YANG CHANGMING ET AL: "Electrically driven microseparation methods for pesticides and metabolites: III. Capillary electrochromatography with novel silica-based stationary phases having a surface-bound surfactant moiety" ELECTROPHORESIS, vol. 21, no. 10, June 2000 (2000-06), pages 1977-1984, XP002468349 ISSN: 0173-0835

## Description

### BACKGROUND OF THE INVENTION

### 1. Field of the Invention

The present invention relates to a method of separating a microorganism using a nonplanar solid substrate and a device for the separation of a microorganism.

### 2. Description of the Related Art

Conventionally, a microorganism is separated form the microorganism-containing media by centrifugation and filtration. In addition, to concentrate or separate a specific cell, the cell is specifically bound to a substrate containing a recipient or ligand, which selectively binds the specific cell, and then, the bound specific cell is concentrated or separated. For example, a sample that contains the specific cell is contacted with a substrate. An antibody that binds to a specific protein of the cell is bound to the substrate, so that the specific cell is bound to the antibody and unbound cells can be washed off, which is an affinity chromatography technique.

The term "a cell" or "a microorganism" refers to at least one cell or microorganism.

Korean Laid-open Patent Publication No.2006-0068979 discloses a device for separating a cell using an ultrasound field and a traveling wave dielectrophoresis. The device includes a piezoelectric transducer which is connected to ends of an upper glass substrate. The transducer converts an external electric input into a mechanical vibration to apply the converted mechanical vibration to the upper glass substrate. The device further comprises a cell separation device in which "n" number of electrodes are disposed on a lower substrate parallel to the upper glass substrate. Each electrode is perpendicular to a lengthwise direction of the piezoelectric transducer, and the "n" number of electrodes are disposed at constant intervals in the lengthwise direction of the piezoelectric transducer.

EP 1 712 284 A discloses a method of and an apparatus for separating cells or viruses in a sample using a hydrophobic solid support, comprising contacting a solution containing cells or viruses with said hydrophobic solid support having a water contact angle between 70° and 90°. In a preferred embodiment, the pH of the solution containing cells or viruses, is in the range of 2.5 to 7, preferably in the range of 2.5 to 4.

EP 1 726 641 A relates to a kit for and a method of separating cells or viruses from a mixture comprising: suspending a sample containing cells or viruses in a kosmotropic salt solution having a pH of 3 to 6; aggregating the cells or viruses by adding a cationic polymer to the suspension; adhering the cells or viruses-polymer complex to a solid substrate by phase separation of cationic polymers; and separating the solid substrate on which the cells or viruses are adhered from the suspension.

US 2002/042125 A1 concerns a device and method for separating an analyte from a fluid sample. The analyte is separated from the sample fluid by introducing the sample into a cartridge having a sample port and a first flow path extending from the sample port to an extraction chamber containing a solid support for capturing the analyte from the sample. The extraction chamber may be a microfabricated chip with an inlet port, an outlet port, and an extraction chamber having internal attachment surfaces, such as a plurality of pillars, having sufficiently high surface area and binding affinity with the target analyte to capture the analyte as the fluid sample flows through the chamber.

WO 2005/093065 A discloses a method for the isolation of nucleic acid from a sample containing cells or complex biological material. The method comprises the steps (i) mixing said sample with a suspension comprising a buffer and a solid support, said suspension having a pH value below pH 5, characterized in that said buffer causes lysis of said cells or said biological material, wherein subsequent to lysis, the nucleic acid released is bound to said solid support; (ii) washing the generated nucleic acid/solid support complex; and (iii) eluting the nucleic acid from the solid support.

WO 98/51693 A refers to a kit for and a method of isolating nucleic acids from a sample of cells, comprising: binding cells in said sample to a solid support to isolate cells from the sample; lysing the isolated cells; and binding nucleic acid released from said lysed cells to said solid support. The solid support may be particulate, preferably comprising magnetic beads. For non-specific cell-binding, the solid support may be functionally coated, for example, positively or negatively charged, hydrophilic or hydrophobic.

However, these conventional techniques are based on fixing a ligand or recipient to a solid substrate or using an external operation power to selectively concentrate or separate a specific cell. Accordingly, a method or device of separating a cell using characteristics of a solid substrate itself and conditions of a liquid medium is not known.

### SUMMARY OF THE INVENTION

The present invention provides a method of separating a microorganism using a nonplanar solid substrate: as defined in claim 1.

The present invention also provides a device for the separation of a microorganism including a nonplanar solid substrate according to claim 11.

### BRIEF DESCRIPTION OF THE DRAWINGS

The above and other features and advantages of the present invention will become more apparent by describing in detail exemplary embodiments thereof with reference to the attached drawings in which:
FIG. 1 is a bar chart illustrating the effects of a buffer on the binding of *E.coli* to a solid substrate having a surface with a pillar array of a fluidic device;
FIG. 2 is a bar chart illustrating the effects of the salt concentration of a liquid medium on the binding of *E.coli* to a solid substrate having a surface with a pillar array of a fluidic device;
FIG. 3 is a bar chart illustrating the effects of the pH of a liquid medium and of surface materials on the binding of *E.coli* contained in a blood sample to a solid substrate having a surface with a pillar array of a fluidic device;
FIG. 4 depicts optical microscopic images showing a surface with a pillar array of a solid substrate of a fluidic device, after a blood sample (pH 5.2) diluted in an acetate buffer is contacted with the solid substrate (upper image) and then washed with 100 mM sodium acetate (pH 4.0) (lower image);
FIG. 5 shows the effects of the pH of a diluted urine sample on the binding of *E.coli* to a solid substrate;
FIG. 6 is a bar chart illustrating the effects of pH and of the surface material on the binding of *E.coli* contained in a diluted urine sample to a solid substrate having a surface with a pillar array;
FIG. 7 is a bar chart illustrating the results of separating a cell from a urine sample containing the cell using a solid substrate having a surface with a pillar array of a fluidic device with respect to urine variation;
FIG. 8 is a bar chart illustrating the results of separating *E.coli* from a urine sample containing *E.coli* using a solid substrate having a surface with a pillar array with respect to the urine dilution ratio;
FIG. 9 is a bar chart illustrating the results of separating *E.coli* from a urine sample containing *E.coli* using a solid substrate having a surface with a pillar array with respect to components added to the urine sample; and
FIG. 10 is a bar chart illustrating the results of concentrating *E.coli* contained in a urine sample using a solid substrate having a surface with a pillar array with respect to the urine dilution ratio and to the flow rate of the sample.

### DETAILED DESCRIPTION OF THE INVENTION

The present invention will now be described in more detail with reference to the accompanying drawings, in which exemplary embodiments of the invention are shown.

A method of separating a microorganism according to an embodiment of the present invention includes contacting a microorganism-containing sample with a nonplanar solid substrate in a liquid medium having a pH of 3.0 to 6.0. By the contacting, the microorganism is bound to the solid substrate.

In a system comprising a liquid medium that is in contact with a solid substrate, a microorganism, such as bacteria, can be present in the liquid medium or can be bound to the solid substrate. The location of microorganism may be determined by a difference in the surface tensions of the liquid medium and the microorganism. For example, when the liquid medium has a greater surface tension than the microorganism, the microorganism may be easily bound to a solid substrate having low surface tension, that is, to a hydrophobic solid substrate; when the liquid medium has a lower surface tension than the microorganism, the microorganism may be easily bound to a solid substrate having high surface tension, that is, to a hydrophilic solid substrate; and when a liquid medium and a microorganism have nearly the same surface tension, the surface tension does not affect the binding of a microorganism to the solid substrate and other interaction factors, such as electrostatic interaction, may affect such binding (see Applied and Environmental Microbiology, July 1983, p.90-97). In addition, it is known that microorganisms can be bound to a solid substrate by electrostatic attraction as well as by a thermodynamic approach based on the surface tension. However, such bindings occur very slowly and the bound quantity is very small.

Inventors of the present invention made efforts to solve these problems of the conventional techniques and found that a microorganism can be separated in a high yield by contacting a nonplanar solid substrate with a microorganism-containing sample in the pH of 3.0 to 6.0. The high yield may result from the increased surface area of the solid substrate. Further, by using a liquid medium of pH 3.0 to 6.0, the cell membrane of a microorganism is denatured and thus the solubility of a microorganism in a liquid medium is lowered and more microorganisms tend to bind to the solid surface. However, the present invention is not limited to such a mechanism.

During the contacting process, the sample can be any sample containing a microorganism. For example, the sample can be a biological sample containing a microorganism, a clinical sample containing a microorganism, or a lab sample containing a microorganism. In the present specification, the biological sample refers to a sample that includes or is formed from a cell or tissue, such as a cell or biological liquid separated from an individual. The individual can be an animal including humans. The biological sample can be saliva, sputum, blood, blood cell (for example, red blood cell or white blood cell), amniotic fluid, serum, semen, bone marrow, tissue or micro needle biopsy sample, urine, peritoneum fluid, pleura fluid, or cell cultures. In addition, the biological sample can be a tissue section, such as a frozen section taken for a histological object. Preferably, the biological sample is a clinical sample derived from a human patient. More preferably, the biological sample is blood, urine, saliva, or sputum.

According to the present invention, a microorganism that is to be separated can be a bacterial cell, fungus, or a virus.

During or prior to the contacting process, the biological sample is diluted in a liquid medium. The liquid medium can be a solution or buffer that buffers the liquid medium at a low pH. The buffer can be a phosphate buffer, such as sodium phosphate of pH 3.0 to 6.0, or an acetate buffer, such as sodium acetate of pH 3.0 to 6.0. The degree of dilution is 1:1 to 1:10.

During the contacting process, the liquid medium may have a salt concentration of 10 mM to 500 mM, and preferably, of 50 mM to 300 mM. That is, the sample is contacting the nonplanar solid substrate in a liquid medium having an acetate or phosphate ion concentration of 10 mM to 500 mM, preferably of 50 mM to 300 mM. The above described preferred pH-range and salt concentrations refer to the pH and salt concentrations of the liquid medium containing the cell-containing sample during the contacting step.

During the contacting process, the solid substrate has a nonplanar shape so that the surface area of the solid substrate can be increased compared to a plane.

The nonplanar solid substrate is selected from a solid substrate having a surface with a plurality of pillars, a bead-shaped solid substrate, and a sieve-shaped solid substrate having a plurality of pores at its surface. The solid substrate can be a single solid substrate or a combination of solid substrates, such as a solid substrate assembly which fills a tube or container.

During the contacting process, the solid substrate may form an inner wall of a microchannel or microchamber of a microfluidic device. Accordingly, the method according to an embodiment of the present invention can be used in a fluidic device or microfluidic device having at least one inlet and outlet connected through a channel or microchannel.

As used herein, the term "microfluidic device" incorporates the concept of a microfluidic device that comprises microfluidic elements such as, e.g., microfluidic channels (also called microchannels or microscale channels). As used herein, the term "microfluidic" refers to a device component, e.g., chamber, channel, reservoir, or the like, that includes at lest one cross-sectional dimension, such as depth, width, length, diameter, etc. of from about 0.1 micrometer to about 1000 micrometer. Thus, the term "microchamber" and "microchannel" refer to a channel and a chamber that includes at lest one cross-sectional dimension, such as depth, width, and diameter of from about 0.1 micrometer to about 1000 micrometer, respectively.

In a method according to an embodiment of the present invention, during the contacting process, the nonplanar solid substrate may have a surface having a plurality of pillars. A method of forming pillars on a solid substrate is well known in the art. For example, micro pillars can be formed in a high density using a photolithography process used in a semiconductor manufacturing process. The pillars may have an aspect ratio, the height of the pillar : the length of the cross section of 1:1-20:1, but the aspect ratio is not limited thereto. The term "aspect ratio" used herein refers to a ratio of a height of a pillar to the length of a cross section of a pillar. The length of a cross section of the pillar refers to a diameter when the shape of the cross section is a circle and it refers to an average value of the length of each side when the shape of the cross section is a rectangle. In the pillar structure, a ratio of the pillar height to the distance between adjacent pillars may be in the range of 1:1 to 25:1. The distance between adjacent pillars may be in the range of 5 µm to 100 µm.

In the method, during the contacting process, the nonplanar solid substrate or at least one defined surface region thereof may have a water contact angle of 70° to 95°. The property of the solid substrate having a water contact angle of 70° to 95° is obtained by coating any of octadecyldimethyl(3-trimethoxysilyl propyl)ammonium (TMSAC) or tridecafluorotetrahydrooctyltrimethoxysilane (FTEOS) or CF₃(CF₂)₃CH₂CH₂Sl(OCH₃) ₃, CF₃ (CF₂)₅CH₂CH₂Sl(OCH₃)₃, CF₃(CF₂)₇CH₂CH₂Sl(OCH₃)₃, CF₃ (CF₂)₉CH₂CH₂SI(OCH₃)₃, (CF₃)₂CF(CF₂)₄CH₂CH₂SI(OCH₃)₃, (CF₃) ₂CF(CF₂)₆CH₂CH₂Sl(OCH₃)₃, (CF₃)₂CF(CF₂)₈CH₂CH₂Sl(OCH₃)₃, CF₃ (C₆H₄)C₂H₄Si(OCH₃)₃, CF₃ (CF₂)₃ (C₆H₄)C₂H₄Si(OCH₃)₃, CF₃ (CF₂)₅(C₆H₄)C₂H₄Si(OCH₃)₃, CF₃ (CF₂)₇(C₆H₄)C₂H₄Si(OCH₃)₃, CF₃ (CF₂) ₃CH₂CH₂SiCH₃ (OCH₃)₂, CF₃ (CF₂)₅CH₂CH₂SiCH₃ (OCH₃)₂, CF₃ (CF₂)₇CH₂CH₂SiCH₃(OCH₃)₂, CF₃(CF₂)₉CH₂CH₂SiCH₃(OCH₃)₂, (CF₃) ₂CF(CF₂)₄CH₂CH₂SiCH₃ (OCH₃)₂, (CF₃)₂CF(CF₂)₆CH₂CH₂SiCH₃ (OCH₃)₂, (CF₃) ₂CF(CF₂)₈CH₂CH₂SiCH₃ (OCH₃)₂, CF₃ (C₆H₄)C₂H₄SiCH₃ (OCH₃)₂, CF₃ (CF₂)₃ (C₆H₄)C₂H₄SiCH₃ (OCH₃)₂, CF₃ (CF₂)₅(C₆H₄)C₂H₄SiCH₃ (OCH₃)₂, CF₃ (CF₂)₇(C₆H₄)C₂H₄SiCH₃(OCH₃)₂, CF₃(CF₂)₃CH₂CH₂Si(OCH₂CH₃)₃, CF₃ (CF₂)₅CH₂CH₂Si(OCH₂CH₃)₃, and/or CF₃ (CF₂)₇CH₂CH₂Si(OCH₂CH₃) onto the surface or defined areas of the surface of the solid substrate. For example, a SiO₂ layer of a solid support may be coated with a self-assembled monolayer (SAM) of a compound selected from the group consisting ofTMSAC and FTEOS to provide a water contact angle of 70-95°.

In this application, the term "water contact angle" refers to water contact angle measured by a Kruss Drop Shape Analysis System type DSA 10 Mk2. A droplet of 1.5 µl deionized water is automatically placed on the sample. The droplet was monitored every 0.2 seconds for a period of 10 seconds by a CCD-camera and analyzed by Drop Shape Analysis software (DSA version 1.7, Kruss). The complete profile of the droplet was fitted by the tangent method to a general conic section equation. The angles were determined both at the right and left side. An average value is calculated for each drop and a total of five drops per sample are measured. The average of the five drops is taken the contact angle.

In the method, during the contacting process, the nonplanar solid substrate may have at least one amine-based functional group, which is positively charged at a pH of 3.0 to 6.0 at its surface or surface regions thereof. The surface with the amine-based functional group is obtained by coating polyethyleneiminetrimethoxysilane (PEIM) onto a solid substrate or defined regions thereof. For example, the coated surface can be obtained by self-assembled molecule (SAM) coating polyethyleneiminetrimethoxysilane (PEIM) on a SiO₂ layer of the solid substrate. The amine-based functional group is positively charged at a pH of 3.0-6.0.

For example, the solid substrate can be formed of glass, silicon wafer, plastic, or the like, but is not limited thereto. When a solid substrate having the surface having the water contact angle of 70° to 95° and/or a surface having at least one amine-based functional group is contacted with a sample containing a microorganism in a liquid medium, the microorganism is assumed to be bound to the solid substrate. However, the present invention is not limited to such a specific mechanism.

The method may further include, after the contacting process, washing the nonplanar solid substrate and removing unbound matters, i.e. other materials excluding the target microorganism, which are not bound to the solid substrate. During the washing process, any solution that does not release the target microorganism bound to the solid substrate from the solid substrate and does removes impurities, which may adversely affect subsequent processes, can be used. For example, an acetate buffer or phosphate buffer which is used as a binding buffer can be used as the washing solution. The washing solution may have the pH of 3.0 to 6.0.

In the present specification, "separation of a microorganism" intends to include concentrating a microorganism in the sample as well as purely separating the microorganism.

The concentrated microorganism bound to the nonplanar solid substrate according to an embodiment of the present invention can be consecutively used in an additional process, such as DNA separation, without separating the microorganism from the solid substrate. Alternatively, the concentrated microorganism bound to the solid substrate can be eluted from the nonplanar solid substrate, and then, the eluted microorganism is used in an additional process.

Accordingly, the method according to an embodiment of the present invention may further include eluting of the bound microorganism, after the contacting and/or the washing. In the eluting process, an eluting solution used can be any solution known in the art suitable for releasing the microorganism from the nonplanar solid substrate. For example, the eluting solution can be water or tris buffer. The pH of the eluting solution may be 6.0 or higher.

A device for separating a microorganism according to an embodiment of the present invention includes a container which includes a nonplanar solid substrate, a sample inlet, and a buffer solution storage unit.

In the device, the solid substrate has a nonplanar surface having a greater surface area in relation to a planar surface.

The nonplanar solid substrate can be selected from a solid substrate having a surface with a plurality of pillars, a bead-shaped solid substrate, and a sieve-shaped solid substrate having a plurality of pores at its surface. The solid substrate can be a single solid substrate or a combination of solid substrates, such as a solid substrate assembly which fills a tube or container.

In the device, the nonplanar solid substrate may form an inner wall of a microchannel or microchamber of a microfluidic device. Accordingly, the device can be a fluidic device or microfluidic device having at least one inlet and outlet connected through a channel or microchannel.

In the device, the nonplanar solid substrate may have a surface having a plurality of pillars. A method of forming pillars on a solid substrate is well known in the art. For example, micro pillars can be formed in a high density using a photolithography process used in a semiconductor manufacturing process. The pillars may have an aspect ratio, the height of the pillar : the length of the cross section of 1:1-20:1, but the aspect ratio is not limited thereto. The term "aspect ratio" used herein refers to a ratio of a height of a pillar to the length of a cross section of a pillar. The length of a cross section of the pillar refers to a diameter when the shape of the cross section is a circle and it refers to an average value of the length of each side when the shape of the cross section is a rectangle. In the pillar structure, a ratio of the pillar height to the distance between adjacent pillars may be in the range of 1:1 to 25:1. The distance between adjacent pillars may be in the range of 5 µm to 100 µm.

In the device, the nonplanar solid substrate has a water contact angle of 70° to 95°. The property of the solid substrate having a water contact angle of 70° to 95° may be provided by coating a surface of the non-planar solid support with a compound selected from the group consisting of octadecyldimethyl (3-trimethoxysilyl propyl)ammonium (TMSAC), tridecafluorotetrahydrooctyltrimethoxysilane (FTEOS), CF₃(CF₂)₃CH₂CH₂SI(OCH₃) ₃, CF₃(CF₂)₅CH₂CH₂SI(OCH₃)₃, CF₃(CF₂)₇CH₂CH₂SI(OCH₃)₃, CF₃ (CF₂)₉CH₂CH₂SI(OCH₃)₃, (CF₃)₂CF(CF₂)₄CH₂CH₂SI(OCH₃)₃, (CF₃) ₂CF(CF₂)₆CH₂CH₂SI(OCH₃)₃, (CF₃)₂CF(CF₂)₈CH₂CH₂SI(OCH₃)₃, CF₃ (C₆H₄)C₂H₄Si(OCH₃)₃, CF₃ (CF₂)₃ (C₆H₄)C₂H₄Si(OCH₃)₃, CF₃ (CF₂)₅(C₆H₄)C₂H₄Si(OCH₃)₃, CF₃ (CF₂)₇(C₆H₄)C₂H₄Si(OCH₃)₃, CF₃ (CF₂) ₃CH₂CH₂SiCH₃(OCH₃)₂, CF₃(CF₂)₅CH₂CH₂SiCH₃(OCH₃)₂, CF₃ (CF₂)₇CH₂CH₂SiCH₃ (OCH₃)₂, CF₃(CF₂)₉CH₂CH₂SiCH₃(OCH₃)₂, (CF₃) ₂CF(CF₂)₄CH₂CH₂SiCH₃ (OCH₃)₂, (CF₃)₂CF(CF₂)₆CH₂CH₂SiCH₃ (OCH₃) ₂, (CF₃) ₂CF(CF₂)₈CH₂CH₂SiCH₃ (OCH₃)₂, CF₃ (C₆H₄)C₂H₄SiCH₃ (OCH₃)₂, CF₃ (CF₂) ₃ (C₆H₄)C₂H₄SiCH₃ (OCH₃)₂, CF₃ (CF₂)₅(C₆H₄)C₂H₄SiCH₃ (OCH₃)₂, CF₃ (CF₂)₇(C₆H₄)C₂H₄SiCH₃ (OCH₃)₂, CF₃ (CF₂)₃CH₂CH₂Si(OCH₂CH₃) ₃, CF₃ (CF₂)₅CH₂CH₂Si(OCH₂CH₃)₃, and CF₃ (CF₂)₇CH₂CH₂Si(OCH₂CH₃). For example, a SiO₂ layer of a solid support may be coated with a self-assembled monolayer (SAM) of a compound selected from the group consisting of TMSAC and FTEOS to provide a water contact angle of 70-95°.

In the device, the nonplanar solid substrate may have at least one amine-based functional group at its surface. The substrate or regions thereof may be coated with a compound selected from the group consisting of polyethyleneiminetrimethoxysilane (PEIM), aminopropyltriethoxysilane, N-(3-trimethoxysily)-propyl)ethylenediamine, and N-trimethoxysilylpropy-N,N,N-chloride trimethylammonium to provide a surface having the at least one amine-based functional group. For example, a SiO2 layer of a solid support may be coated with an SAM of PEIM. The amine-based functional group is positively charged at a pH of 3.0-6.0.

In the device, the nonplanar solid substrate can be a substrate formed of any material or that is coated with a material that has the water contact angel in the range described above an/or that has at least one amine-based functional group at its surface. For example, the solid substrate can be formed of glass, can be a silicon wafer, plastic, or the like, but is not limited thereto. When a solid substrate having a surface having the water contact angle of 70° to 95° and/or a surface having at least one amine-based functional group is contacted with a sample containing a microorganism in a liquid medium, the microorganism is assumed to be bound to the solid substrate. However, the present invention is not limited to such a specific mechanism.

In the device, the container comprising the solid substrate may have various shapes, and can be a chamber, a channel, or a column. The device may include a column filled with bead-shaped solid substrates.

In the device, the container may include a microorganism sample inlet and a buffer storage unit. The buffer storage unit may contain a buffer which adjusts the liquid media to a pH of 3.0 to 6.0 in the contacting step and/or dilutes the sample. The buffer can be an acetate buffer or a phosphate buffer. The sample inlet and the buffer storage unit are in fluid communication with the inside of the container.

The present invention will be described in further detail with reference to the following examples. These examples are for illustrative purposes only and are not intended to limit the scope of the present invention.

### Example

### Example 1: Effects of the Buffer and the Buffer Concentration on the Capturing of Bacteria using a Solid Substrate having a Pillar Array of Fluidic Device

In the current example, a bacteria-containing sample was flowed through a fluidic device comprising an inlet, an outlet, and a chamber having a pillar array on a 10 mm x 23 mm silicon chip substrate so that the bacterial cell was bound to the nonplanar solid substrate. The cell number (Nᵢₙ) in the bacteria-containing sample introduced into the chamber and the cell number (Nₒᵤₜ) of the sample discharged from the chamber were determined by colony counting. Then, the bacteria capturing efficiency (%), (Nᵢₙ - Nₒᵤₜ)/Nᵢₙ)x100, was determined with respect to the buffer and the respective buffer salt concentration used. In the pillar array, the distance between adjacent pillars was 12 µm, the height of each pillar was 100 µm, and the sectional surface of each pillar was a regular square with each side of 25 µm.

The pillar array had a surface coated with TMSAC having a water contact angle of 70° to 95°, more specifically, 80°.

The bacteria sample used was prepared by suspending an *E.coli* sample of 1.0 OD₆₀₀ in a LB medium using 1x PBS of pH 7.0 and then diluting the suspended sample with respective buffers to 100 times. As a result, a sample of 0.01 OD₆₀₀ was obtained. The sample was adjusted to a pH of 4.0 by using 100 mM sodium phosphate buffer (pH 4.0), 100 mM sodium acetate buffer (pH 4.0), 100 mM sodium citrate buffer (pH 4.0), and 300 mM sodium phosphate buffer (pH 4.0). 200 µℓ of each of the adjusted samples was conveyed at a flow rate of 300 µℓ/minute from the inlet through the chamber to the outlet. This experiment was repeated three times.

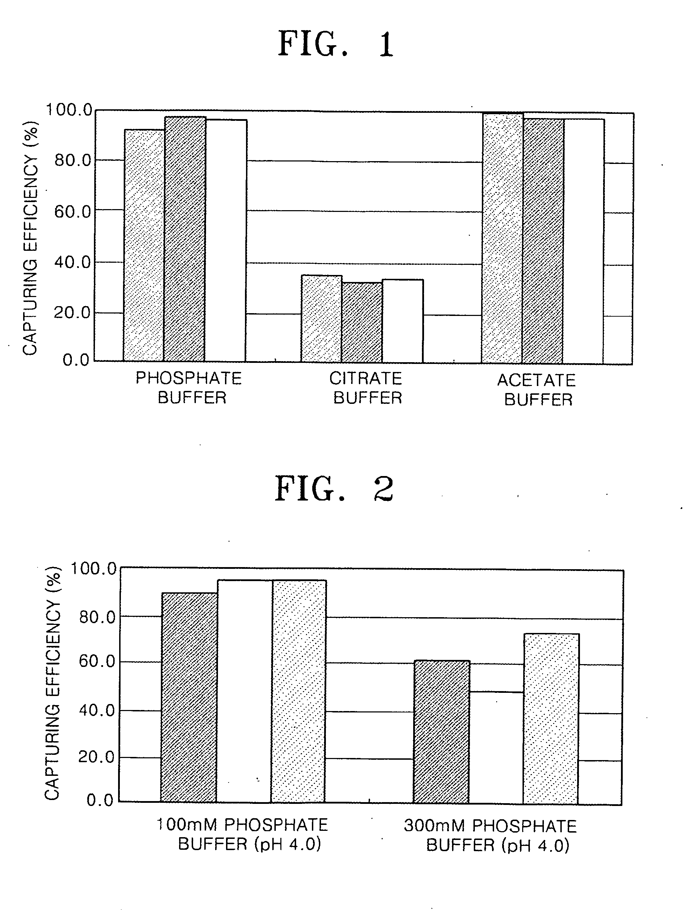

FIG. 1 is a bar chart illustrating the effects of the buffer on the capturing efficiency of binding *E.coli* to the solid substrate having a surface with a pillar array of a fluidic device. As illustrated in FIG. 1, among the buffers having a low pH and similar salt concentrations, the sodium phosphate buffer and the sodium acetate buffer showed excellent capturing efficiency > 90 %.

FIG. 2 is a bar chart illustrating the effects of the salt concentration of the buffer on the capturing efficiency for binding *E.coli* to a solid substrate having a surface with a pillar array of a fluidic device. As illustrated in FIG. 2, the lower the salt concentration was, the higher the capturing efficiency of the bacterial cell to the solid substrate. However, a real sample, such as a biological sample, requires a buffer solution having a buffering capacity to maintain the pH of the liquid medium in the range of 3.0 to 6.0. In consideration of such characteristics of the real sample, the salt concentration required to obtain high capturing efficiency of the cell to the solid substrate may be in the range of 10 mM to 500 mM, more preferably, 50 mM to 300 mM (not shown in data.)

### Example 2: Capturing of Bacterial Cell in Blood using a Solid Substrate having a Pillar Array of a Fluidic Device

In the current example, a bacteria-containing sample was flowed through a fluidic device comprising an inlet, an outlet, and a chamber having a pillar array on a 10 mm x 23 mm silicon chip substrate so that the bacterial cell was bound to the nonplanar solid substrate. The cell number (Nᵢₙ) in the bacteria-containing sample introduced into the chamber and the cell number (Nₒᵤₜ) of the sample discharged from the chamber were determined by colony counting. Then, the bacteria capturing efficiency (%), (Nᵢₙ - Nₒᵤₜ)/Nᵢₙ)x100, was determined with respect to the surface material of the solid substrate.

In the pillar array, the distance between adjacent pillars was 12 µm, the height of each pillar was 100 µm, and the sectional surface of each pillar was a regular square with each side of 25 µm.

A pillar array having a SiO₂ layer at its surface, a pillar array having a SiO₂ layer coated with PEIM at its surface, a pillar array having a SiO₂ layer coated with TMSAC at its surface, and a pillar array having a SiO₂ layer coated with FTEOS at its surface were prepared.

10 µℓ of 1.0 OD₆₀₀ *E.coli* suspended in 1x PBS (pH 7.0) was added to 990 µℓ of a solution which consisted of 495µℓ of blood and 495µℓ of buffer (pH 3.0, pH 7.0) to prepare a diluted blood sample of 0.01 OD₆₀₀.

200 µℓ of the diluted blood sample was conveyed at a flow rate of 200 µℓ/minute from the inlet through the chamber to the outlet. Then, 300 µℓ of 100 mM sodium acetate (pH 4.0) was conveyed at a flow rate of 200 µℓ/minute from the inlet through the chamber to the outlet, to wash the sample. This experiment was repeated three times.

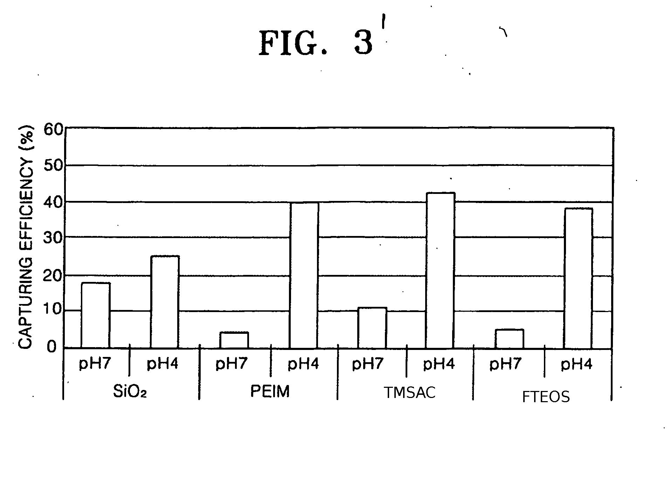

FIG. 3 is a bar chart illustrating the effects of the buffer pH and of the surface material of the pillar array on the capturing efficiency for binding *E.coli* contained in blood to a solid substrate having a surface with a pillar array of a fluidic device. As illustrated in FIG. 3, a sample comprising a liquid media having a low pH showed a higher capturing efficiency to the solid substrate than a sample in a liquid media with a pH of about 7.0. The effects of surface material of the solid substrate on the binding efficiency were lower than the effect of the buffer pH. However, when compared the low pH experiments, a relatively hydrophobic surface having a relatively high electrostatic properties or a low surface tension, such as PEIM, TMSAC and FTEOS, showed a higher capturing efficiency than the SiO₂ surface having a low electrostatic properties and a high surface tension.

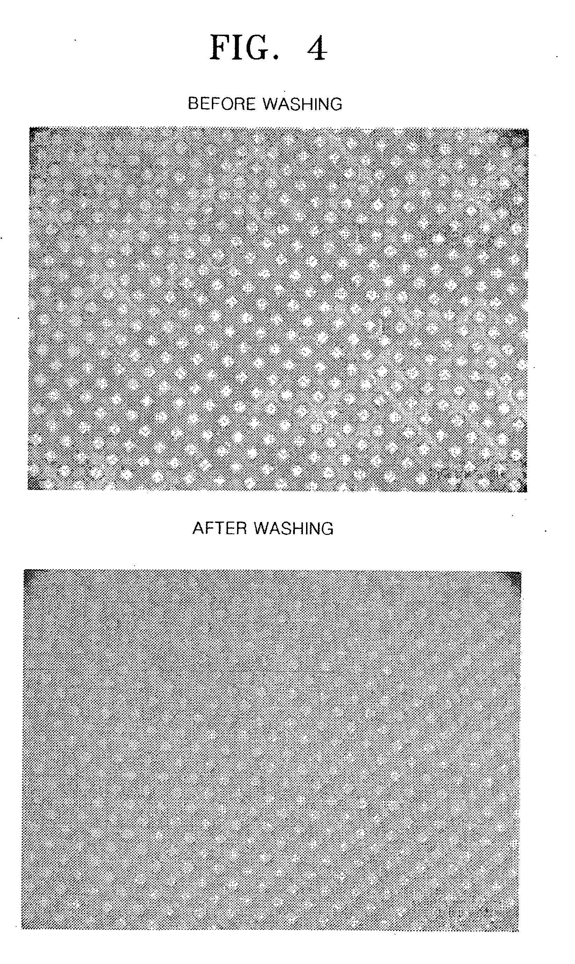

FIG. 4 depicts optical microscopic images of a surface with a pillar array of a solid substrate of a fluidic device. The upper image shows the surface after a blood sample (pH 5.2) diluted in acetate buffer was contacted with the solid substrate. The lower image shows the result after washing the nonplanar solid substrate with 100 mM sodium acetate (pH 4.0) consecutively to the binding. When quantified by colony counting, *E.coli* cell was not eluted in the washing step. In addition, as illustrated in FIG. 4, removal of an animal cell, such as a red blood cell, which can act as a PCR inhibitor during the washing process, can be identified with amicroscopy. In FIG 4A, impurity materials shown between retangular structures is considered to animal cell such as red blood cell and white blood cell, and/or proteins. FIG 4B. shows such materials except bacteria can be removed by a washing process. The method of present invention is directed to capture target bacterial cell after removing the materials. FIG 4. shows that the removal of animal cells such as red blood cell and/or white blood cell, but not bacterial cell, can be identified with a microscopy.

### Example 3: Binding of Bacterial Cell contained in Urine to a Planar Solid Substrate

In the current example, 25.4 mm x 25.4 mm silicon chips containing 24 unit cells respectively including a SiO₂ layer, a SiO₂ layer coated with PEIM, a SiO₂ layer coated with TMSAC, and a SiO₂ layer coated with FTEOS were contacted with a sample including a bacterial cell so that the bacterial cell was bound to the solid substrate and washed. The binding of the bacterial cell was identified using a microscope.

10 µℓ of 1.0 OD₆₀₀ *E.coli* suspended in 1x PBS (pH 7.0) was added to 990 µℓ of a solution which consisted of 495 µℓ of urine and 495 µℓ of buffer (pH 3.0, pH 7.0) to prepare a diluted urine sample of 0.01 OD₆₀₀ sample.

The diluted urine sample was added to the substrate covered with lid in the form of a patch and then incubated for 5 minutes. Then, the liquid media was removed from the substrate and a washing solution was added to the substrate. The substrate was incubated for 5 min in the washing solution for washing the planar solid substrate. When the sodium acetate buffer was used as the liquid medium in the binding step, the sample was washed using the sodium acetate buffer for 5 minutes; and when the sodium phosphate buffer was used as the liquid medium in the binding step, the sample was washed using the sodium phosphate buffer.

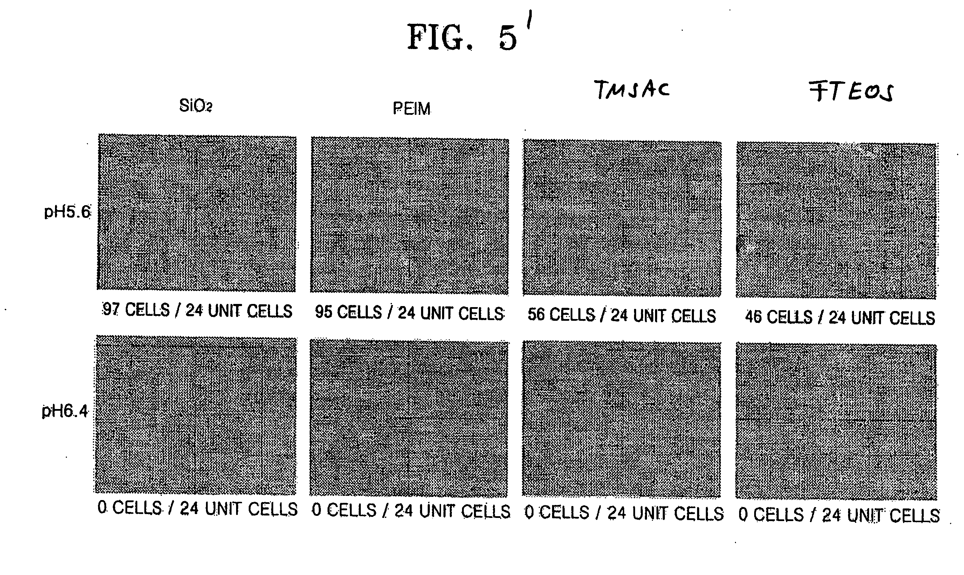

FIG. 5 shows the effects of the pH of the diluted urine sample on the capturing efficiency for binding *E.coli* contained in the sample to a solid substrate. As illustrated in FIG. 5, the pH of the diluted urine sample significantly affected the binding of *E.coli* to the solid substrate. In addition, it was found that *E.coli* was not bound to the solid substrate when the pH is 6.0 or higher. Accordingly, it is desired to perform separating of the bacterial cell in the pH of 6.0 or lower. Specifically, as shown in Fig. 5, the number of E.coli cells attached to the solid substate having a SiO2 layer, a SiO2 layer coated with PEIM, a Si02 layer coated with TMSAC, or a SiO2 layer coated with FTEOS was about 97 cells, 95 cells, 56 cells, and 46 cells, respectively per 25.4 mm x 25.4 mm (24 unit cells) silicon chips at pH 5.6 (upper panel), however, no E.coli cells were remain attached to the same solid substrate at pH 6.4 (lower panel). The number of E.coli cells attached to the solid substrate was determined by counting on the microscopy.

### Example 4: Capturing of Bacterial Cell in Urine using a Solid Substrate having a Surface with a Pillar Array

In the current example, a bacteria-containing sample was flowed through a fluidic device comprising an inlet, an outlet, and a chamber having a pillar array on a 10 mm x 23 mm silicon chip substrate so that the bacterial cell was bound to the nonplanar solid substrate. The cell number (Nᵢₙ) in the bacteria-containing sample introduced into the chamber and the cell number (Nₒᵤₜ) of the sample discharged from the chamber were determined by colony counting. Then, the bacteria capturing efficiency (%), (Nᵢₙ - Nₒᵤₜ)/Nᵢₙ)x100, was determined with respect to the surface material of the solid substrate. In the pillar array, the distance between adjacent pillars was 12 µm, the height of each pillar was 100 µm, and the sectional surface of each pillar was a regular square with each side of 25 µm.

A pillar array having a SiO₂ layer at its surface and a pillar array having a SiO₂ layer coated with PEIM at its surface were prepared.

10 µℓ of 1.0 OD₆₀₀ *E.coli* suspended in 1x PBS (pH 7.0) was added to 990 µℓ of a solution which consisted of 495 µℓ of urine and 495 µℓ of buffer (pH 3.0, pH 7.0) to prepare a diluted urine sample of 0.01 OD₆₀₀ sample.

200 µℓ of the diluted urine sample was conveyed at a flow rate of 200 µℓ/minute from the inlet through the chamber to the outlet. Then, 300 µℓ of 100 mM sodium acetate (pH 4.0) was conveyed at a flow rate of 200 µℓ/minute from the inlet through the chamber to the outlet, to wash the sample. This experiment was repeated three times.

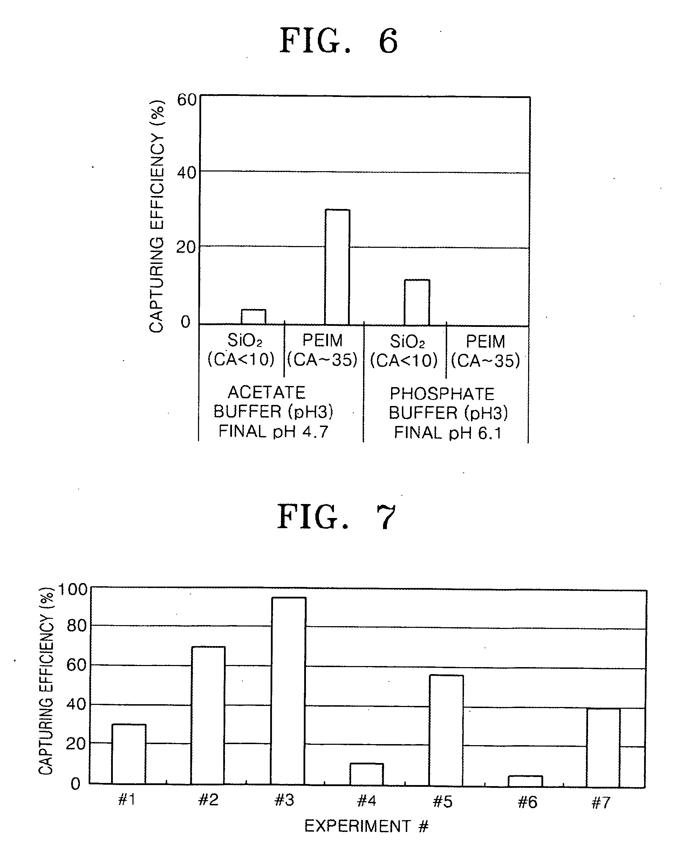

FIG. 6 is a bar chart illustrating the effects of pH and the surface material on the capturing efficiency for binding *E.coli* contained in a diluted urine sample to a solid substrate having a surface with a pillar array. As illustrated in FIG. 6, when the pH is 4.7, the capturing efficiency of *E.coli* was high. The cell capturing efficiency measured according to the current example is shown in Table 1:

**Table 1**

| Diluted Buffer and Final pH | Acetate Buffer (pH3) Final pH 4.7 | | Phosphate Buffer (pH3) Final pH 6.1 | |
|---|---|---|---|---|
| Surface Characteristics (Water Contact Angle: CA) | SiO₂ (<10°) | PEIM (Approximately 35°) | SiO₂ (<10°) | PEIM (Approximately 35°) |
| Capturing Efficiency (%) | 4 | 30 | 11 | 0 |

### Example 5: Capturing of Bacterial Cell in Urine using a Solid Substrate having a Surface with a Pillar Array: Concentration Effects with respect to Urine Variation

In the current example, a bacteria-containing sample was flowed through a fluidic device comprising an inlet, an outlet, and a chamber having a pillar array on a 10 mm x 23 mm silicon chip substrate so that the bacterial cell was bound to the nonplanar solid substrate. The cell number (Nᵢₙ) in the sample introduced into the chamber and the cell number (Nₒᵤₜ) of the sample discharged from the chamber were determined by colony counting. Then, the cell capturing efficiency (%), (Nᵢₙ - Nₒᵤₜ)/Nᵢₙ)x100, was determined with respect to urine used. In the pillar array, the distance between adjacent pillars was 12 µm, the height of each pillar was 100 µm, and the sectional surface of each pillar was a regular square with each side of 25 µm.

A pillar array had a SiO₂ layer coated with PEIM at its surface.

10 µℓ of 1.0 OD₆₀₀ *E.coli* suspended in 1x PBS (pH 7.0) was added to 990 µℓ of a solution which consisted of 495 µℓ of urine and 495 µℓ of buffer (pH 3.0, pH 7.0) to prepare a diluted urine sample of 0.01 OD₆₀₀ sample.

200 µℓ of the diluted urine sample was conveyed at a flow rate of 200 µℓ/minute from the inlet through the chamber to the outlet. Then, 300 µℓ of 100 mM sodium acetate (pH 4.0) was conveyed at a flow rate of 200 µℓ/minute from the inlet through the chamber to the outlet, to wash the sample. The capturing efficiency was calculated by determining the cell number (Nᵢₙ) in the urine sample introduced into the chamber and the cell number (Nₒᵤₜ) of the urine sample discharged from the chamber by colony counting.

FIG. 7 is a bar chart illustrating the results of separating cells from a urine sample containing the cell using the solid substrate having a surface with a pillar array of a fluidic device with respect to urine variation. As illustrated in FIG. 7, the cell capturing efficiency was significantly changed according to a kind of the urine. This may result from the fact that the salt concentration of urine varies according to the body conditions and the alimentation of the person, which provides the urine, and other components. However, the present invention is not limited to such a specific mechanism. In addition, it can be assumed that the pH and the conductivity of the urine are not directly related to a change in the capturing efficiency (not shown in data).

### Example 6: Capturing of Bacterial Cell in Urine using a Solid Substrate having a Surface with a Pillar Array: Concentration Effects with respect to the Dilution and the Washing of Urine

In the current example, a bacteria-containing sample was flowed through a fluidic device comprising an inlet, an outlet, and a chamber having a pillar array on a 10 mm x 23 mm silicon chip substrate so that the bacterial cell was bound to the nonplanar solid substrate. The cell number (Nᵢₙ) in the sample introduced into the chamber and the cell number (Nₒᵤₜ) of the sample discharged from the chamber were determined by colony counting. Then, the cell capturing efficiency (%), (Nᵢₙ - Nₒᵤₜ)/Nᵢₙ)x100, was determined with respect to the dilution rate of the urine. In the pillar array, the distance between adjacent pillars was 12 µm, the height of each pillar was 100 µm, and the sectional surface of each pillar was a regular square with each side of 25 µm.

A pillar array having a SiO₂ layer coated with PEIM at its surface and a pillar array having a SiO₂ layer coated with TMSAC were prepared.

Cell samples of three dilution ratios were prepared, namely, a½ diluted sample of pH 3.97, which was prepared by mixing urine containing *E.coli* of 0.01 OD₆₀₀ 1:1 with 100 mM sodium acetate buffer (pH 3.0) (hereinafter, referred to as 1/2 diluted sample), a 1/5 diluted sample of pH 4.05, which was prepared by mixing urine containing *E.coli* of 0.01 OD₆₀₀- 1:4 with 100 mM sodium acetate buffer (pH 4.0) (hereinafter, referred to as 1/5 diluted sample, and a 1/7 diluted sample of pH 4.05, which was prepared by mixing urine containing *E.coli* of 0.01 OD₆₀₀- 1:6 with 100 mM sodium acetate buffer (pH 4.0) in 1:6 (hereinafter, referred to as 1/7 diluted sample).

200 µℓ of each of these diluted urine samples was conveyed at a flow rate of 200 µℓ/minute from the inlet through the chamber to the outlet. Then, 300 µℓ of 100 mM sodium acetate (pH 4.0) was conveyed from the inlet through the chamber to the outlet at a flow rate of 200 µℓ/minute, for washing. This experiment was performed three times. The capturing efficiency was calculated by determining the cell number (Nᵢₙ) in the urine sample introduced into the chamber and the cell number (Nₒᵤₜ) of the urine sample discharged from the chamber by colony counting.

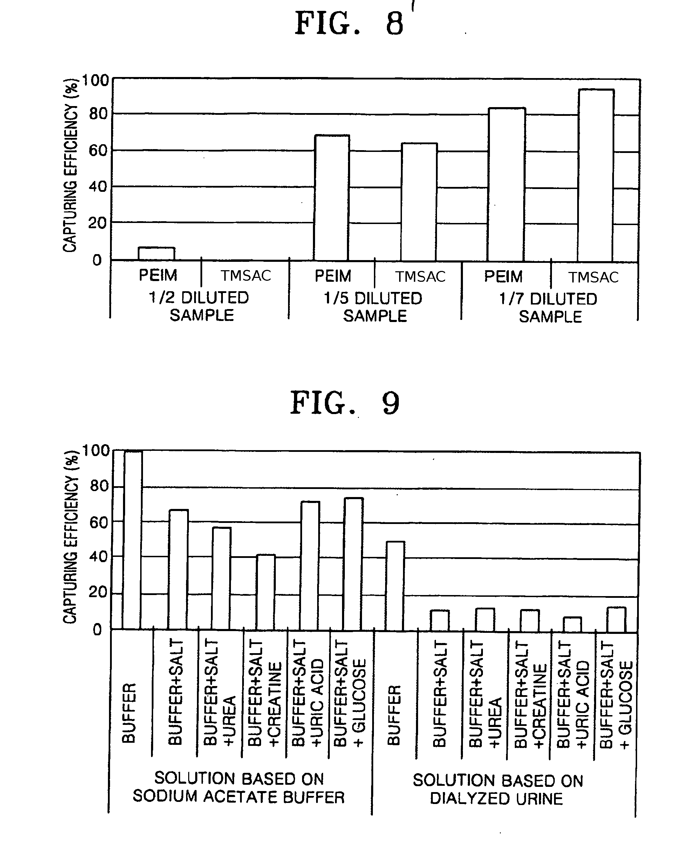

FIG. 8 is a bar chart illustrating the results of concentrating *E.coli* from a urine sample containing *E.coli* using the solid substrate having a surface with a pillar array with respect to ratio of urine dilution. As illustrated in FIG. 8, the higher the dilution ratio of the urine sample was, the higher the cell capturing efficiency. In the washing process, the cells were not eluted.

### Example 7: Capturing E.coli in a Mimic Urine Solution using a Solid Substrate having a Surface with a Pillar Array: Detecting of Factor that inhibits Cell Capturing

In the current example, a bacteria-containing sample was flowed through a fluidic device comprising an inlet, an outlet, and a chamber having a pillar array on a 10 mm x 23 mm silicon chip substrate so that the bacterial cell was bound to the nonplanar solid substrate. The cell number (Nᵢₙ) in the sample introduced into the chamber and the cell number (Nₒᵤₜ) of the sample discharged from the chamber were determined by colony counting. Then, the cell capturing efficiency (%), (Nᵢₙ - Nₒᵤₜ)/Nᵢₙ)x100, was determined. In the pillar array, the distance between adjacent pillars was 12 µm, the height of each pillar was 100 µm, and the sectional surface of each pillar was a regular square with each side of 25 µm.

A pillar array having a SiO₂ layer coated with TMSAC at its surface and a pillar array having a SiO₂ layer coated with SAM were prepared.

As a cell sample, a solution containing *E.coli* of 0.01 OD₆₀₀ were used. The solution was either based on a sodium acetate buffer or on dialyzed urine as the liquid medium.

The solutions based on a sodium acetate buffer (pH 4.0) are as follows:
buffer: 100 mM sodium acetate buffer (pH 4.0);
buffer+salt: a solution containing 88 mM NaCl, 67 mM KCI, 38 mM NH₄Cl, and 18 mM Na₂SO₄ prepared by adding 0.514 g of NaCl, 0.5 g of KCI, 0.203 g of NH₄Cl, and 0.259 g of Na₂SO₄ to 100 mM sodium acetate buffer (pH 4.0);
buffer+salt+urea: a solution that consists of the "buffer+salt" solution and 333 mM urea;
buffer+salt+urea+creatine: a solution that consists of the "buffer+salt" solution, 333 mM urea, and 9.8 mM creatine;
buffer+salt+urea+creatine+uric acid: a solution that consists of the "buffer+salt" solution, 333 mM urea, 9.8 mM creatine, and 2.5 mM uric acid; and
buffer+salt+urea+creatine+uric acid+glucose: a solution that consists of the "buffer+salt" solution, 333 mM urea, 9.8 mM creatine, 2.5 mM uric acid, and 0.6 mM glucose.

The solutions based on dialyzed urine were obtained by mixing dialyzed urine 1:1 with the respective 2x sodium acetate buffer-based solution. The solutions based on dialyzed urine have a final pH of 3.97.

200 µℓ of the urine sample was conveyed at a flow rate of 200 µℓ/minute from the inlet through the chamber to the outlet. This experiment was repeated for three times. The capturing efficiency was calculated by determining the cell number (Nᵢₙ) in the urine sample introduced into the chamber and the cell number (Nₒᵤₜ) of the urine sample discharged from the chamber by colony counting.

FIG. 9 is a bar chart illustrating the results of concentrating the cell in the urine sample using the solid substrate having a surface with a pillar array. As illustrated in FIG. 9, the capturing efficiency was lowered when the urine sample contained salt and other materials, such as creatine.

### Example 8: Capturing E.coli in Urine using a Solid Substrate having a Surface with a Pillar Array: Capturing efficiency with respect to the Dilution Rate of Urine and the Flow rate

In the current example, a bacteria-containing sample was flowed through a fluidic device comprising an inlet, an outlet, and a chamber having a pillar array on a 10 mm x 23 mm silicon chip substrate so that the bacterial cell was bound to the nonplanar solid substrate. The cell number (Nᵢₙ) in the sample introduced into the chamber and the cell number (Nₒᵤₜ) of the sample discharged from the chamber were determined by colony counting. Then, the cell capturing efficiency (%), (Nᵢₙ - Nₒᵤₜ)/Nᵢₙ)x100, was determined. In the pillar array, the distance between adjacent pillars was 12 µm, the height of each pillar was 100 µm, and the sectional surface of each pillar was a regular square with each side of 25 µm.

A pillar array having a SiO₂ layer coated with PEIM by SAM coating process at its surface prepared.

The samples were prepared by mixing 100mM sodium acetate buffer with urine in dilution rates of ½, 1/3, ¼, 1/5 and 1/7 until the final volume of the mixture was 1 ml. Then, 10 µℓ of 1.0 OD₆₀₀ *E.coli* was added to the diluted sample.

200 µℓ of the diluted urine sample was conveyed at a flow rate of 200 µℓ/minute from the inlet through the chamber to the outlet. This experiment was repeated three times. The capturing efficiency was calculated by determining the bacteria cell number (Nᵢₙ) in the urine sample introduced into the chamber and the bacteria cell number (Nₒᵤₜ) of the urine sample discharged from the chamber by colony counting.

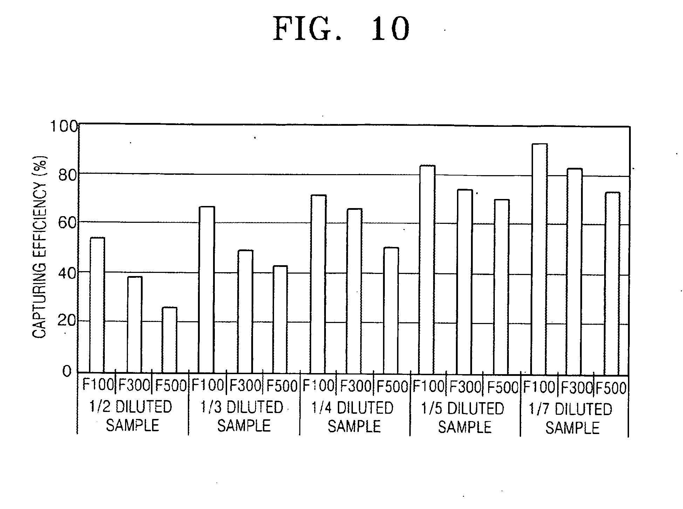

FIG. 10 is a bar chart illustrating the results of concentrating *E.coli* contained in the respective urine sample using the solid substrate having a surface with a pillar array with respect to the urine dilution ratio and the flow rate of conveying the sample through the chamber. As illustrated in FIG. 10, the more diluted the urine sample was, the higher the cell capturing efficiency . Further, if the flow rate increased, the cell capturing efficiency decreased.

According to a method of separating a microorganism, a microorganism, such as a bacterial cell, fungus cell, or virus, present in a biological sample, can be efficiently separated.

By using a device for the separation of a microorganism, a microorganism, such as a bacterial cell, fungus cell, or virus, present in a biological sample, can be efficiently separated.

While the present invention has been particularly shown and described with reference to exemplary embodiments thereof, it will be understood by those of ordinary skill in the art that various changes in form and details may be made therein without departing from the scope of the present invention as defined by the following claims.

## Claims

1. A method of separating a microorganism comprising contacting a nonplanar solid substrate with a microorganism-containing sample in a liquid medium having a pH of 3.0 to 6.0, wherein the microorganism-containing sample is diluted in a ratio of 1:1 to 1:10 with the liquid medium, which liquid medium is preferably a phosphate buffer or an acetate buffer, wherein the nonplanar solid substrate is selected from the group consisting of a solid substrate having a surface comprising a pillar structure formed of a plurality of pillars, a bead-shaped solid substrate, and a sieve-shaped solid substrate having a surface comprising pores, and wherein the nonplanar solid substrate or at least one defined surface region thereof has a water contact angle of 70° to 95° or has at least one amine-based functional group, which amine-based functional group is positively charged at a pH of 3.0 to 6.0, at its surface, and wherein the surface or the at least one defined surface region of the nonplanar solid substrate is coated with octadecyldimethyl(3-trimethoxysilyl propyl)ammonium (TMSAC) or tridecafluorotetrahydrooctyltrimethoxysilane (FTEOS) or polyethyleneiminetrimethoxysilane (PEIM).

2. The method according to claim 1, wherein the microorganism is bacteria, fungus, or a virus.

3. The method according to claim 1 or 2, wherein the microorganism-containing sample is a biological sample.

4. The method according to claim 3, wherein the biological sample is blood, urine, or saliva.

5. The method according to any of claims 1 to 4, wherein the liquid medium has a salt concentration of 10 mM to 500 mM.

6. The method according to claim 5, wherein the liquid medium has a salt concentration of 50 mM to 300 mM.

7. The method according to claim 1, wherein each pillar of a solid substrate having a surface comprising a pillar structure has an aspect ratio of 1:1 to 20:1, wherein the aspect ratio is height of a pillar : the length of the cross-section.

8. The method according to claim 1 or 7, wherein the nonplanar solid substrate comprises a solid substrate having a surface comprising a pillar structure, wherein the ratio of the pillar height to the distance between adjacent pillars is in the range of 1:1 to 25:1.

9. The method according to any of claims 1, 7 or 8, wherein the nonplanar solid substrate comprises a solid substrate having a surface comprising a pillar structure, wherein the distance between adjacent pillars is in the range of 5 µm to 100 µm.

10. The method according to any of claims 1 to 9 further comprising, after the contacting step, washing the nonplanar solid substrate and removing unbound matters, which are not bound to the nonplanar solid substrate.

11. A device for the separation of a microorganism, the device comprising a container which comprises a nonplanar solid substrate, a sample inlet, and a buffer solution storage unit containing a phosphate or an acetate buffer which adjusts a liquid media containing a sample to a pH of 3.0 to 6.0 and dilutes the sample, wherein the sample inlet and the buffer solution storage unit are in fluid communication with the inside of the container, wherein the nonplanar solid substrate is selected from the group consisting of a solid substrate having a surface comprising a pillar structure formed of a plurality of pillars, a bead-shaped solid substrate, and a sieve-shaped solid substrate having a surface comprising pores, and wherein the nonplanar solid substrate or at least one defined surface region thereof has a water contact angle of 70° to 95° or has at least one amine-based functional group, which amine-based functional group is positively charged at a pH of 3.0 to 6.0, at its surface.

12. The device according to claim 11, wherein each pillar of the solid substrate having a surface comprising a pillar structure has an aspect ratio of 1:1 to 20:1, wherein the aspect ratio is height of a pillar : the length of the cross-section.

13. The device according to claim 11 or 12, wherein the nonplanar solid substrate comprises a solid substrate having a surface comprising a pillar structure, wherein the ratio of the pillar height to the distance between adjacent pillars is in the range of 1:1 to 25:1.

14. The device according to any of claims 11 to 13, wherein the nonplanar solid substrate comprises a solid substrate having a surface comprising a pillar structure, wherein the distance between adjacent pillars is in the range of 5 µm to 100 µm.

15. The device according to claim 11, wherein the surface or the at least one defined region of the nonplanar solid substrate is coated with octadecyldimethyl(3-trimethoxysilyl propyl)ammonium (TMSAC) or tridecafluorotetrahydrooctyltrimethoxysilane (FTEOS).

16. The device according to claim 11, wherein the surface or the at least one defined region of the nonplanar solid substrate is coated with polyethyleneiminetrimethoxysilane (PEIM).

## Patentansprüche

1. Verfahren zur Trennung eines Mikroorganismus umfassend das Inkontakbringen eines nichtplanaren festen Substrats mit einer einen Mikroorganismus enthaltenden Probe in einem flüssigen Medium mit einem pH-Wert von 3,0 bis 6,0, wobei die Mikroorganismus enthaltende Probe in einem Verhältnis von 1:1 bis 1:10 mit dem flüssigen Medium verdünnt wird, wobei das flüssige Medium vorzugsweise ein Phosphatpuffer oder ein Acetatpuffer ist, wobei das nichtplanare feste Substrat gewählt ist aus der Gruppe bestehend aus einem festen Substrat mit einer Oberfläche, welche eine Säulenstruktur umfasst, gebildet von einer Vielzahl von Säulen, einem kugelförmigen festen Substrat und einem siebförmigen festen Substrat mit einer Oberfläche, welche Poren umfasst, und wobei das nichtplanare feste Substrat oder wenigstens ein definierter Oberflächenbereich dessen einen Wasserkontaktwinkel von 70° bis 95° aufweist oder wenigstens eine auf Amin basierende funktionelle Gruppe an der Oberfläche aufweist, wobei die auf Amin basierende funktionelle Gruppe bei einem pH-Wert von 3,0 bis 6,0 positiv geladen ist, und wobei die Oberfläche oder wenigstens ein definierter Oberflächenbereich des nichtplanaren festen Substrats mit Oktadecyldimetyl(3-trimethoxysilylpropyl)amonium (TMSAC) oder Tridecafluortetrahydrooctoyltrimethoxisylan (FTEOS) oder Polyethylenimintrimethoxysilan (PEIM) beschichtet ist.

2. Verfahren nach Anspruch 1, wobei der Mikroorganismus Bakterie, Pilz oder ein Virus ist.

3. Verfahren nach Anspruch 1 oder 2, wobei die Mikroorganismen enthaltende Probe eine biologische Probe ist.

4. Verfahren nach Anspruch 3, wobei die biologische Probe Blut, Urin oder Speichel ist.

5. Verfahren nach einem der Ansprüche 1 bis 4, wobei das flüssige Medium eine Salzkonzentration von 10 mM bis 500 mM aufweist.

6. Verfahren nach Anspruch 5, wobei das flüssige Medium eine Salzkonzentration von 50 mM bis 300 mM aufweist.

7. Verfahren nach Anspruch 1, wobei jede Säule eines festen Substrats, welches eine Oberfläche aufweist, die eine Säulenstruktur umfasst, ein Aspektverhältnis von 1:1 bis 20:1 aufweist, wobei das Aspektverhältnis die Höhe einer Säule : die Länge des Querschnittes ist.

8. Verfahren nach Anspruch 1 oder 7, wobei das nichtplanare feste Substrat ein festes Substrat umfasst, mit einer Oberfläche umfassend eine Säulenstruktur, wobei das Verhältnis der Säulenhöhe zu dem Abstand zwischen benachbarten Säulen in dem Bereich von 1:1 bis 25:1 liegt.

9. Verfahren nach einem der Ansprüche 1, 7 oder 8, wobei das nichtplanare feste Substrat ein festes Substrat umfasst, mit einer Oberfläche umfassend eine Säulenstruktur, wobei der Abstand zwischen benachbarten Säulen in dem Bereich von 5 µm bis 100 µm liegt.

10. Verfahren nach einem der Ansprüche 1 bis 9, des Weiteren umfassend das Waschen des nichtplanaren festen Substrats und Entfernens ungebundener Materie, welche nicht an das nichtplanare feste Substrat gebunden ist, nach dem Schritt des Inkontaktbringens.

11. Einrichtung zur Trennung eines Mikroorganismus, wobei die Einrichtung einen Container umfasst, welcher ein nichtplanares festes Substrat umfasst, einen Probeneinlass und eine Pufferlösungspeichereinheit enthaltend ein Phosphat oder ein Acetatpuffer, welcher ein flüssiges Medium, das eine Probe enthält, auf einen pH-Wert von 3,0 bis 6,0 einstellt und die Probe verdünnt, wobei der Probeneinlass und die Pufferlösungspeichereinheit in Fluidverbindung mit dem Inneren des Behälters stehen, wobei das nichtplanare feste Substrat gewählt ist aus der Gruppe bestehend aus einem festen Substrat mit einer Oberfläche, welche eine Säulenstruktur umfasst, die aus einer Vielzahl von Säulen gebildet ist, einem kugelförmigen festen Substrat und einem siebförmigen festen Substrat, welches eine Oberfläche aufweist, die Poren umfasst, und wobei das nichtplanare feste Substrat oder wenigstens ein definierter Oberflächenbereich dieser einen Wasserkontaktwinkel von 70° bis 95° aufweist oder wenigstens eine auf Amin basierende funktionelle Gruppe an der Oberfläche umfasst, wobei die auf Amin basierende funktionelle Gruppe bei einem pH-Wert von 3,0 bis 6,0 positiv geladen ist.

12. Einrichtung nach Anspruch 11, wobei jede Säule des festen Substrats, welches eine Oberfläche aufweist, die eine Säulenstruktur umfasst, ein Aspektverhältnis von 1:1 bis 20:1 aufweist, wobei das Aspektverhältnis die Höhe einer Säule : die Länge des Querschnitts ist.

13. Einrichtung nach Anspruch 11 oder 12, wobei das nichtplanare feste Substrat ein festes Substrat umfasst, mit einer Oberfläche umfassend eine Säulenstruktur, wobei das Verhältnis der Säulenhöhe zu dem Abstand der benachbarten Säulen in dem Bereich von 1:1 1 bis 25:1 liegt.

14. Einrichtung nach einem der Ansprüche 11 bis 13, wobei das nichtplanare feste Substrat ein festes Substrat umfasst, mit einer Oberfläche umfassend eine Säulenstruktur, wobei der Abstand zwischen benachbarten Säulen in dem Bereich von 5 µm bis 100 µm liegt.

15. Einrichtung nach Anspruch 11, wobei die Oberfläche oder wenigstens ein definierter Bereich des nichtplanaren festen Substrats mit Oktadecyldimetyl (3-trimethoxysilylpropyl)amonium (TMSAC) oder Tridecafluortetrahydrooctyltrimethoxysilan (FTEOS) beschichtet ist.

16. Einrichtung nach Anspruch 11, wobei die Oberfläche oder wenigstens ein definierter Bereich des nichtplanaren festen Substrats mit Polyetylenimintrimethoxysilan (PEIM) beschichtet ist.

## Revendications

1. Procédé de séparation d'un micro-organisme comprenant la mise en contact d'un substrat solide non planaire contenant un échantillon contenant un micro-organisme dans un milieu liquide d'un pH de 3,0 à 6,0, où l'échantillon contenant un micro-organisme est dilué dans un rapport de 1:1 à 1:10 avec le milieu liquide, lequel milieu liquide est de préférence un tampon de phosphate ou un tampon d'acétate, où le substrat solide non planaire est sélectionné parmi le groupe consistant en un substrat solide ayant une surface comprenant une structure en piliers composée d'une pluralité de piliers, d'un substrat solide en forme de perle et d'un substrat solide en forme de tamis présentant une surface comprenant des pores, et où le substrat solide non planaire ou au moins une région superficielle définie de celui-ci comporte un angle de contact avec l'eau de 70° à 95° ou bien présente au moins un groupe fonctionnel à base d'amine lequel groupe fonctionnel à base d'amine est chargé positivement à un pH de 3,0 à 6,0 à sa surface, et où la surface ou bien ladite au moins une région superficielle définie du substrat solide non planaire est enduite d'octadécyldiméthyle(3-triméthoxysilyle propyle)ammonium (TMSAC) ou tridécafluorotétrahydrooctyltriméthoxysilane (FTEOS) ou polyéthylèneiminetriméthoxysilane (PEIM).

2. Procédé selon la revendication 1, où le micro-organisme est une bactérie, un champignon ou un virus.

3. Procédé selon la revendication 1 ou 2, où l'échantillon contenant un micro-organisme est un échantillon biologique.

4. Procédé selon la revendication 3, où l'échantillon biologique est du sang, de l'urine ou de la salive.

5. Procédé selon l'une quelconque des revendications 1 à 4, où le milieu liquide présente une concentration en sel de 10 mM à 500 mM.

6. Procédé selon la revendication 5, où le milieu liquide présente une concentration en sel de 50 mM à 300 mM.

7. Procédé selon la revendication 1, où chaque pilier d'un substrat solide présentant une surface comprenant une structure en piliers à un rapport d'aspect de 1:1 à 20:1, où le rapport d'aspect correspond à la hauteur du pilier sur la longueur de la section transversale.

8. Procédé selon la revendication 1 ou 7, où le substrat solide non planaire comprend un substrat solide ayant une surface comprenant une structure en piliers où le rapport de la hauteur de pilier à la distance séparant des piliers adjacents est compris dans la fourchette allant de 1:1 à 25:1.

9. Procédé selon l'une quelconque des revendications 1, 7 ou 8, où le substrat solide non planaire comprend un substrat solide ayant une surface comprenant une structure en piliers où la distance séparant des piliers adjacents est comprise dans la fourchette allant de 5 µm à 100 µm.

10. Procédé selon l'une quelconque des revendications 1 à 9 comprenant en outre, après la phase de mise en contact, le lavage du substrat solide non planaire et d'élimination des matières non liées, qui ne sont pas liées au substrat solide non plan.

11. Dispositif pour la séparation d'un micro-organisme, le dispositif comprenant un contenant présentant un substrat solide non planaire, une entrée d'échantillon et une unité de stockage de solution tampon contenant un tampon de phosphate ou un tampon d'acétate qui ajuste un milieu liquide contenant un échantillon à un pH de 3,0 à 6,0 et dilue l'échantillon, où l'entrée d'échantillon et l'unité de stockage de solution tampon sont en communication fluide avec l'intérieur du contenant, où le substrat solide non planaire est sélectionné parmi le groupe consistant en un substrat solide ayant une surface comprenant une structure en piliers composée d'une pluralité de piliers, d'un substrat solide en forme de perle et d'un substrat solide en forme de tamis présentant une surface comprenant des pores, et où le substrat solide non planaire ou au moins une région superficielle définie de celui-ci comporte un angle de contact avec l'eau de 70° à 95° ou bien présente au moins un groupe fonctionnel à base d'amine lequel groupe fonctionnel à base d'amine est chargé positivement à un pH de 3,0 à 6,0 à sa surface.

12. Dispositif selon la revendication 11, où chaque pilier du substrat solide ayant une surface comprenant une structure en piliers présente un rapport d'aspect de 1:1 à 20:1, où le rapport d'aspect correspond à la hauteur du pilier sur la longueur de la section transversale.

13. Dispositif selon la revendication 11 ou 12, où le substrat solide non planaire comprend un substrat solide ayant une surface comprenant une structure en piliers où le rapport de la hauteur de pilier à la distance séparant des piliers adjacents est compris dans la fourchette allant de 1:1 à 25:1.

14. Dispositif selon l'une des revendications 11 à 13, où le substrat solide non planaire comprend un substrat solide ayant une surface comprenant une structure en piliers où la distance séparant des piliers adjacents est comprise dans la fourchette allant de 5 µm à 100 µm.

15. Dispositif selon la revendication 11, où la surface ou bien ladite au moins une région superficielle définie du substrat solide non planaire est enduite d'octadécyldiméthyle(3-triméthoxysilyle propyle)ammonium (TMSAC) ou tridécafluorotétrahydrooctyltriméthoxysilane (FTEOS).

16. Dispositif selon la revendication 11, où la surface ou bien ladite au moins une région superficielle définie du substrat solide non planaire est enduite de polyéthylèneiminetriméthoxysilane (PEIM).
